(19) Europäisches Patentamt
European Patent Office
Office européen des brevets

(11) Publication number: **0 336 452 B1**

(12) # EUROPEAN PATENT SPECIFICATION

(45) Date of publication of patent specification: **13.10.93**

(51) Int. Cl.[5]: **C12N 15/52**, C12P 13/22

(21) Application number: **89108165.5**

(22) Date of filing: **16.02.84**

(60) Publication number of the earlier application in accordance with Art.76 EPC: **0 136 359**

The file contains technical information submitted after the application was filed and not included in this specification

(54) **Process for preparing L-phenylalanine.**

(30) Priority: **17.02.83 JP 25397/83**
**17.02.83 JP 25398/83**
**28.05.83 JP 94392/83**
**29.07.83 JP 138775/83**
**04.08.83 JP 142804/83**
**24.09.83 JP 176758/83**

(43) Date of publication of application:
**11.10.89 Bulletin 89/41**

(45) Publication of the grant of the patent:
**13.10.93 Bulletin 93/41**

(84) Designated Contracting States:
**AT BE CH DE FR GB LI NL SE**

(56) References cited:
**EP-A- 0 066 129**
**EP-A- 0 093 611**

(73) Proprietor: **KYOWA HAKKO KOGYO CO., LTD.**
**6-1, Ohtemachi 1-chome**
**Chiyoda-ku Tokyo 100(JP)**

(72) Inventor: **Oka, Tetsuo**
**2360-17, Naramachi**
**Midori-ku**
**Yokohama-shi Kanagawa 227(JP)**
Inventor: **Katsumata, Ryoichi**
**Popuragaoka Coopo 6-401**
**12-3, Naruse 2-chome**
**Machida-shi Tokyo 194(JP)**
Inventor: **Ozaki, Akio**
**6-6, Asahimachi 3-chome**
**Machida-shi Tokyo 194(JP)**
Inventor: **Mizukami, Toru**
**14-10, Asahimachi 2-chome**
**Machida-shi Tokyo 194(JP)**
Inventor: **Yokoi, Haruhiko**
**6-6, Asahimachi 3-chome**
**Machida-shi Tokyo 194(JP)**
Inventor: **Hara, Masako**
**40-11, Sagamigaoka 5-chome**
**Zama-shi Kanagwa 228(JP)**

(74) Representative: **VOSSIUS & PARTNER**
**Postfach 86 07 67**
**D-81634 München (DE)**

**EP 0 336 452 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing L-phenylalanine by a novel method for the expression of a gene. More specifically, the present invention relates to a process for producing L-phenylalanine by transforming a host microorganism belonging to the genus Corynebacterium or Brevibacterium with a recombinant DNA of a DNA fragment containing the gene coding for chorismate mutase and prephenate dehydratase and a vector DNA, culturing the transformant in a nutrient medium, accumulating the L-phenylalanine in the culture medium and recovering it therefrom.

For the direct production of amino acids by fermentation, methods using wild-type strains, or mutant strains such as auxotrophic strains and amino acid analog-resistant strains, of the bacteria belonging to the genus Corynebacterium, Brevibacterium, Serratia, and the like are known.

For example, the production of histidine using a strain resistant to histidine analog (Japanese Patent Publication No. 8596/81), production of tryptophan using a strain resistant to tryptophan analog (Japanese Patent Publication Nos. 4505/72 and 19037/76), production of isoleucine using a strain resistant to isoleucine analog (Japanese Patent Publication Nos. 38995/72, 6237/76 and 32070/79), production of phenylalanine using a strain resistant to phenylalanine analog (Japanese Patent Publication No. 10035/81), production of tyrosine using a strain requiring phenylalanine for its growth or being resistant to tyrosine [Agr. Chem. Soc., Japan 50 (1) R79-R87 (1976)], production of arginine using a strain resistant to L-arginine analog [Agr. Biol. Chem., 36, 1675-1684 (1972), Japanese Patent Publication Nos. 37235/79 and 150381/82] and the like are known.

The present invention relates to the production of L-phenylalanine using a microorganism belonging to the genus Corynebacterium or Brevibacterium by recombinant DNA technology different from the conventional mutational breeding technology for the purpose of improving the amino acid productivity.

The present inventors have constructed plasmid vectors autonomously replicable in a microorganism belonging to the genus Corynebacterium or Brevibacterium and having selectable markers and adequate cloning sites and have developed a highly efficient transformation system (Japanese Published Unexamined Patent Application Nos. 183799/82, 186492/82 and 186489/82). Further, the present inventors have found that the plasmid vectors are useful for expressing a foreign gene in a host microorganism and increasing the productivity of amino acids by ligating a DNA fragment containing a foreign gene involved in the biosynthesis of amino acids such as glutamic acid and lysine to the plasmid vectors according to the procedures in recombinant DNA technology (Methods in Enzymology 68, Recombinant DNA, edited by Ray Wu, Academic Press 1979, U.S. Patent No. 4,237,224) and transforming Corynebacterium glutamicum L-22 or its derivatives using the transformation methods developed by the present inventors (Japanese Published Unexamined Patent Application No. 126789/83).

The present inventors have found that microorganisms prepared by the above method acquire an increased productivity of phenylalanine.

No example of expressing a desired gene and increasing the productivity of L-phenylalanine in a host microorganism belonging to the genus Corynebacterium or Brevibacterium by introducing a recombinant DNA containing such desired gene and vector, one of which is foreign to a host microorganism, into such host microorganism has been reported.

The present invention is explained in detail below.

The present invention provides a process for producing L-phenylalanine by cultivating in a medium a transformant which is obtained by transforming a microorganism belonging to the genus Corynebacterium or Brevibacterium with a recombinant DNA of a DNA fragment containing the gene coding for chorismate mutase and prephenate dehydratase and a vector DNA.

As the DNA fragment containing the gene coding for chorismate mutase and prephenate dehydratase used in the present invention, the DNA fragment containing said gene involved in the biosynthesis of L-phenylalanine derived from eukaryotes, prokaryotes, viruses, bacteriophages or plasmids is used. As the gene derived from prokaryotes, the gene derived from a bacterium belonging to the genus Escherichia, Corynebacterium, Brevibacterium, Microbacterium, Bacillus, Staphylococcus, Streptococcus or Serratia and involved in the biosynthesis of L-phenylalanine of the present invention or the metabolism relating to the biosynthesis is preferably used.

As the most preferable source of the gene, amino acid-producing strains and amino acid analog-resistant strains derived from the bacteria described above are used. The amino acid analog-resistance can be conferred on a plasmid after cloning.

As the gene involved in the metabolism relating to the biosynthesis of phenylalanine, the genes coding for chorismate mutase and prephenate dehydratase are mentioned. Further, the above-mentioned genes on which the phenotype resistant to phenylalanine, tyrosine or an analog thereof such as para-

fluorophenylalanine (PFP) is conferred are used. As the source of such a gene, Corynebacterium glutamicum K38 resistant to PFP is mentioned.

The vector used in the present invention should autonomously replicate in cells of the host microorganism. Preferably, plasmids isolated from microorganisms belonging to the genus Corynebacterium by the present inventors or derivatives thereof such as pCG1 (Japanese Published Unexamined Patent Application No. 134500/82), pCG2 (Japanese Published Unexamined Patent Application No. 35197/83, pCG4 (Japanese Published Unexamined Patent Application No. 183799/82), pCE51, pCE52 (Japanese Published Unexamined Patent Application No. 126789/83), pCE53 (Japanese Published Unexamined Patent Application No. 25398/83), pCE54, pCG11, pCB100 (Japanese Published Unexamined Patent Application No. 105999/83), and the like are mentioned,

Microorganisms carrying these plasmids have been deposited with the Fermentation Research Institute, Agency of Industrial Science and Technology, and the American Type Culture Collection, U.S.A. under the following accession numbers.

| Plasmid | FERM P- | ATCC |
|---|---|---|
| pCG1 | 5865 | 31808 |
| pCG2 | 5954 | 31832 |
| pCG4 | 5939 | 31830 |
| pCE54 | - | 39019 |
| pCG11 | - | 39022 |
| pCB101 | - | 39020 |

Plasmids pCE51, 52, 53 and 54 can be introduced from the plasmids mentioned above as follows.

For example, pCE51 is prepared as follows.

pCG1 is isolated from the cultured cells of Corynebacterium glutamicum 225-57 (FERM P-5865, ATCC 31808) by the method described in the specification of Japanese Published Unexamined Patent Application No. 134500/82. pGA22 is isolated from the cultured cells of Escherichia coli harboring the plasmid by a conventional method [An, G. et al.,: J. Bacteriol., 140, 400 (1979)]. Plasmid pCG1 is linearized with restriction endonuclease BglII and a fragment of pGA22 digested with BamHI and containing kanamycin resistance ($Km^R$) gene is ligated to the linearized pCG1 using the same cohesive ends of both plasmids. Isolation of pCE51 from the ligated DNA mixture is achieved by selecting the transformants belonging to the genus Corynebacterium or Brevibacterium and containing $Km^R$ derived from pGA22, and analyzing the plasmid in the transformant.

pCE51 has a molecular weight of about 6 Kb and cleavage sites for HincII, HindIII, SmaI, XhoI and EcoRI and gives $Km^R$ phenotype.

pCE52 and pCE53 are prepared as follows.

Plasmid pCG1 is isolated from the cultured cells of Corynebacterium glutamicum 225-57 (FERM P-5865, ATCC 31808) by the method described in the above application and plasmid pGA22 is isolated from the cultured cells of Escherichia coli harboring the plasmid by a conventional method. pCG1 with a unique BglII site is linearized with restriction enzyme BglII and pGA22 with two BamHI sites are partially digested with BamHI. The cohesive ends of both plasmids are annealed and ligated with T4 phage DNA ligase to make a composite molecule. Selection of the recombinant plasmids from the ligation mixture is carried out by isolating transformants of the genus Corynebacterium or Brevibacterium on the basis of drug-resistances derived from pGA22 and then analyzing the plasmids in the transformants.

pCE52 and pCE53 have a molecular weight of about 10.9 Kb and cleavage sites for EcoRI, SalI, SmaI and XhoI. While pCE52 gives the phenotypes of resistance to chloramphenicol ($Cm^R$) and $Km^R$, pCE53 gives resistance to tetracycline ($Tc^R$), $Cm^R$ and $Km^R$ phenotypes. Since the cleavage site for XhoI is present in the $Km^R$ gene, selection by insertional inactivation (prevention of the expression of a gene by the insertion of a DNA fragment into the gene) is possible.

Transformation with the ligated DNA mixture is carried out using protoplasts of the genus Corynebacterium or Brevibacterium, and the method described in Japanese Published Unexamined Patent Application Nos. 186492/82 and 186489/82.

Among the genes responsible for drug resistance derived from pGA22, those except for the resistance gene which is insertionally inactivated are used for selection. In no DNA adding system, transformants are recovered as a colony regenerated on a hypertonic agar medium containing generally 0.4 - 1.6 µg/mℓ Tc, 2.5 - 5 µg/mℓ Cm, 100 - 800 µg/mℓ Km, 100 - 400 µg/mℓ Sm or 200 - 1,000 µg/mℓ Spec which does not allow the reversion to normal cells of the recipient protoplasts which are not treated with the ligation

mixture. Alternatively, transformants are regenerated unselectively on a regeneration medium, and the resultant cells are scraped and resuspended, followed by the isolation of those cells grown on an agar medium containing a drug in a concentration wherein thr recipient normal cells can not grow, that is, generally, 0.5 - 4 µg/mℓ Tc, 2 - 15 µg/mℓ Cm, 2 - 25 µg/mℓ Km, 5 - 50 µg/mℓ Sm or 50 - 500 µg/mℓ Spec. Some of the transformants selected by $Tc^R$, $Cm^R$ or $Km^R$ are simultaneously endowed with other drug-resistances derived from plasmid pGA22.

Plasmid DNAs in these transformants can be isolated from cultured cells of the transformants and purified according to the methods described in Japanese Published Unexamined Patent Application Nos. 134500/82 and 186489/82. The structures of the DNAs can be determined by digesting them with various restriction endonucleases and analyzing the DNA fragments by agarose gel electrophoresis. The plasmids isolated from the transformants are pCE51, pCE52 and pCE53. Recovery of plasmids from the strains is carried out according to the methods described in Japanese Published Unexamined Patent Application Nos. 134500/82, 183799/82 and 35197/83. Preparation of a recombinant DNA of a vector DNA with a DNA fragment containing a gene is carried out by conventional in vitro recombinant DNA technology.

In vitro recombinant DNA technology is carried out by cleavage and ligation of a donor DNA containing a desired gene to a vector DNA (refer to Japanese Published Unexamined Patent Application No. 126789/83, USP 4,237,224).

The ligase reaction gives recombinants containing genes other than the desired gene. The desired recombinant DNA can be obtained by directly transforming a microorganism of the genus Corynebacterium or Brevibacterium with the ligated DNA mixture, selecting the transformants having the phenotype derived from the desired gene and isolating the desired recombinant DNA from the cultured cells of the transformants. Instead of cloning the desired gene directly in a microorganism of the genus Corynebacterium or Brevibacterium, the desired gene can be cloned by using another host-vector system such as Escherichia coli. Then, it is recloned in vitro into a vector of the genus Corynebacterium or Brevibacterium to transform these microorganisms and transformants containing the desired recombinant plasmid are selected as mentioned above.

The method of cloning a gene in a host microorganism of Escherichia coli is described in, for example, Methods in Enzymology, 68, Ray Wu (Ed) Academic press New York (1979).

The following references are helpful for the construction of recombinant DNA:

S.N. Cohen, et al., U.S.P. No. 4,237,224;

Idenshi Sosa Jikkenho, edited by Yasuyuki Takagi, printed by Kodansha Scientific (1980);

Methods in Enzymology 68, Recombinant DNA, edited by Ray Wu, Academic Press, 1979

Japanese Published Unexamined Patent Application No. 126789/82.

Microorganisms belonging to the genus Corynebacterium or Brevibacterium and which are competent for incorporating DNAs may be used as the host microorganisms in the present invention. The following are examples of a suitable host microorganism.

| | Accession Number | |
|---|---|---|
| | FERM P- | ATCC |
| Corynebacterium glutamicum | | 13032 |
| Corynebacterium glutamicum L-15 | 5946 | 31834 |
| Corynebacterium glutamicum LA-105 | | |
| Corynebacterium glutamicum K-38 | 7087 | |
| Corynebacterium glutamicum K-43 | 7162 | |
| Corynebacterium herculis | | 13868 |
| Corynebacterium herculis L-103 | 5947 | 31866 |
| Corynebacterium acetoacidophilum | | 13870 |
| Corynebacterium lilium | | 15990 |
| Brevibacterium divaricatum | | 14020 |
| Brevibacterium divaricatum L-204 | 5948 | 31867 |
| Brevibacterium lactofermentum | | 13869 |
| Brevibacterium lactofermentum L-312 | 5949 | 31868 |
| Brevibacterium flavum | | 14067 |
| Brevibacterium immariophilium | | 14068 |
| Brevibacterium thiogenitalis | | 19240 |

Transformation of the host microorganisms with recombinant DNAs is carried out by the following steps:

1) Preparation of protoplasts from cultured cells;

2) Transformation of the protoplasts with a recombinant DNA;

3) Regeneration of the protoplasts to normal cells and selection of a transformant;

These steps are described in detail below.

1. Preparation of protoplasts from cultured cells:

The preparation of protoplasts is carried out by culturing a microorganism under conditions which render it sensitive to lysozyme, a lytic enzyme, and treating the cultured cells with lysozyme in a hypertonic solution to remove the cell wall. In order to render microbial cells sensitive to lysozyme, reagents inhibiting the synthesis of bacterial cell walls are used. For example, microbial cells sensitive to lysozyme are obtained by adding, during the logarithmic growth phase, an amount of penicillin which does not inhibit or sub-inhibits the growth and then continuing culturing for several generations.

For culturing, any medium wherein the microorganism can grow may be used. For example, a nutrient medium NB (pH 7.2) consisting of 20 g/ℓ powdered bouillon and 5 g/ℓ yeast extract and a semi-synthetic medium SSM (pH 7.2) consisting of 10 g/ℓ glucose, 4 g/ℓ $NH_4Cl$, 2 g/ℓ urea, 1 g/ℓ yeast extract, 1 g/ℓ $KH_2PO_4$, 3 g/ℓ $K_2HPO_4$, 0.4 g/ℓ $MgCl_2 \cdot 6H_2O$, 10 mg/ℓ $FeSO_4 \cdot 7H_2O$, 0.2 mg/ℓ $MnSO_4 \cdot$ (4-6)$H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6MO_7O_{24} \cdot 4H_2O$, 30 µg/ℓ biotin and 1 mg/ℓ thiamine hydrochloride are used.

Microbial cells are inoculated in the medium and culturing is carried out with shaking.

The optical density (OD) of the culture medium at 660 nm is monitored with a colorimeter and penicillin, such as penicillin G, is added to the medium at an initial stage of the logarithmic growth phase (OD : 0.1 - 0.4) in a concentration of 0.1 to 2.0 U/mℓ. Culturing is continued to an OD value of 0.3 - 0.5, and then cells are harvested and washed with the SSM medium. The washed cells are resuspended in a suitable hypertonic medium such as PFM medium (pH 7.0 - 8.5) wherein 0.4M sucrose and 0.01M $MgCl_2 \cdot 6H_2O$ are added to 2 fold diluted SSM medium, and RCG medium (pH 7.0 - 8.5) consisting of 5 g/ℓ glucose, 5 g/ℓ casein hydrolysate, 2.5 g/ℓ yeast extract, 3.5 g/ℓ $K_2HPO_4$, 1.5 g/ℓ $KH_2PO_4$, 0.41 g/ℓ $MgCl_2 \cdot 6H_2O$, 10 mg/ℓ $FeSO_4 \cdot 7H_2O$, 2 mg/ℓ $MnSO_4 \cdot$(4-6)$H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6Mo_7O_{24} \cdot 4H_2O$, 30 ug/ℓ biotin, 2 mg/ℓ thiamine hydrochloride and 135 g/ℓ sodium succinate or RCGP medium which consists of RCG medium and 3% polyvinyl pyrrolidone. To the cell suspension, lysozyme is added to a final concentration of 0.2 to 10 mg/mℓ, and the mixture is allowed to react at a temperature of 30 to 37°C. Protoplast formation proceeds with time and is monitored with an optical microscope. The period required for the conversion of most cells to protoplasts depends on the concentrations of the penicillin used for the lysozyme-sensitization and the amount of lysozyme used. The period is 3 - 24 hours under the conditions mentioned above.

Since protoplasts formed are destroyed under hypotonic conditions, the extent of the formation of protoplast is determined indirectly from the number of normal cells surviving under hypotonic conditions. Generally, the ratio of surviving normal cells are kept below $10^{-4}$ per lysozyme-treated normal cell.

The protoplasts are prepared above have colony-forming (regenerating) ability on a suitable hypertonic agar medium. As the agar medium, a nutrient medium, a semi-synthetic medium or a synthetic medium containing various amino acids, which contains 0.3 to 0.8M sodium succinate and 0.5 to 6% polyvinyl pyrrolidone with a molecular weight of 10,000 or 40,000 is preferably used. Generally, a semi-synthetic medium RCGP (pH 7.2) wherein 1.4% agar is added to the RCGP agar medium is used. Regeneration is carried out at a temperature of 25 to 35°C. The cultivation time required for the regenration of protoplasts depends upon the strain used, and usually in 10 to 14 days colonies can be picked up. The efficiency of the regeneration of protoplasts on the RCGP medium also depends on the strain used, the concentrations of the penicillin added during the cultivation and the concentration of lysozyme used. The efficiency is generally $10^{-2}$ - $10^{-4}$ cells per normal cell treated with lysozyme.

2. Transformation of the protoplasts with a recombinant DNA:

Introduction of a recombinant DNA into the protoplast is carried out by mixing the protoplast and the DNA in a hypertonic solution which protects the protoplast and by adding to the mixture polyethyleneglycol (PEG, average molecular weight: 1,540 - 6,000) or polyvinylalcohol (PVA, degree of polymerization: 500 - 1,500) and a divalent metal cation which stimulates the uptake of DNA. As a stabilizing agent for the hypertonic conditions, those generally used to protect protoplasts of other microorganisms such as sucrose and sodium succinate are also employed. PEG and PVA can be used at a final concentration of 5 to 60%

and 1 to 20%, respectively. Divalent metal cations such as $Ca^{++}$, $Mg^{++}$, $Mn^{++}$, $Ba^{++}$ and $Sr^{++}$ are effectively used alone or in combination at a final concentration of 1 to 100 mM. Transformation is carried out satisfactorily at 0 to 25 °C.

3. Regenration of the protoplasts to normal cells and selection of a transformant:

Regeneration of the protoplast transformed with a recombinant DNA is carried out in the same way as mentioned above by spreading the protoplast on a hypertonic agar medium such as RCGP medium containing sodium succinate and polyvinyl pyrrolidone and incubating at a temperature wherein normal cells can grow, generally 25 to 35 °C. Transformants are obtained by selecting the phenotypes derived from donor DNAs. The selection by characteristic phenotype endowed may be carried out simultaneously with regeneration on a hypertonic agar medium or may be carried out on a hypotonic agar medium after non-selective reversion to normal cells on a hypertonic agar medium.

In the case of using the lysozyme-sensitive strains described as the preferred host microorganisms, transformation may be carried out by the steps described in (1) to (3) except that the cultured cells are directly treated with lysozyme without prior treatment with penicillin in step (1). In that case, transformants are obtained at an efficiency of $10^{-2}$ to $10^{-4}$ per regenerated cell.

The following strains are the examples of the transformants obtained by the present invention.

The phenotypic expression of the recombinant DNA is carried out by growing the transformant in a conventional nutrient medium. Appropriate reagents may be added to the medium according to the phenotypes expected from the gene DNA or vector DNA on the recombinant DNA.

The thus obtained transformant is cultured in a conventional manner used in the production of amino acids by fermentation. That is, the transformant is cultured in a conventional medium containing carbon sources, nitrogen sources, inorganic materials, amino acids, vitamines, etc. under aerobic conditions, with adjustment of temperature and pH. Amino acids, thus accumulated in the medium are recovered.

As the carbon source, various carbohydrates such as glucose, glycerol, fructose, sucrose, maltose, mannose, starch, starch hydrolyzate and molasses, polyalcohols and various organic acids such as pyruvic acid, fumaric acid, lactic acid and acetic acid may be used. According to the assimilability of the microorganism strain used, hydrocarbon and alcohols are employed. Blackstrap molasses is most preferably used.

As the nitrogen source, ammonia, various inorganic or organic ammonium salts as ammonium chloride, ammonium sulfate, ammonium carbonate and ammonium acetate, urea, and nitrogenous organic substances such as peptone NZ-amine meat extract, yeast extract, corn steep liquor, casein hydrolyzate, fish meal or its digested product, defatted soybean or its digested product and chrysalis hydrolyzate are appropriate.

As the inorganic materials, potassium iihydrogenphosphate, dipotassium hydrogenphosphate, ammonium sulfate, ammonium chloride, magnesium sulfate, sodium chloride, ferrous sulfate, manganese sulfate and calcium carbonate may be used. Vitamines and amino acids required for the growth of microorganisms may not be added, provided that they are supplied with other components mentioned above.

Culturing is carried out under aerobic conditions with shaking or aeration-agitation. Culturing temperature is preferably 20 to 40 °C. The pH of the medium during culturing is maintained around neutral. Culturing is continued until a considerable amount of an amino acid is accumulated, generally for 1 to 5 days.

After completion of the culturing, cells are removed and the amino acid is recovered from the culture liquor by conventional manners such as treatment with active carbon or ion exchange resin.

In spite of the high similarity in microbiological characteristics, so called glutamic acid-producing microorganisms which produce glutamic acid in large amounts are classified into various species and even into different genera such as Corynebacterium and Brevibacterium, which is probably because of their industrial importance. However, it has been pointed out that these microorganisms should belong to one species because of nearly the same composition of amino acids in the cell wall and the base composition of DNAs. Recently, it has been reported that these microoganisms have at least 70 to 30% homology in DNA-DNA hybridization, indicating that these microoganisms are closely related. See, e.g., Komatsu Y.: Report of the Fermentation Research Institute, No. 55, 1 (1980), and Suzuki, K., Kaneko, T., and Komagata, K.: Int. J. Syst. Bacterial., 31, 131 (1981).

In consideration of the facts mentioned above, it is readily assumed that the usefulness of the present invention is applicable to all the glutamic acid-producing microorganisms. In order to stably maintain recombinant DNA molecules and express the DNA in these species, slight differences of such properties of the host microorganisms as homology in the DNA are negligible and it is sufficient for host microorganisms

to allow the autonomous replication of plasmids and expression of genes on them. That these microorganisms have such abilities is apparent from the facts that plasmid pCG4 which was isolated from Corynebacterium glutamicum 225-250 (Japanese Published Unexamined Patent Application No. 183799/82) and having an streptomycin and/or spectinomycin resistant gene could replicate in the glutamic acid-producing microorganisms such as those belonging to the genus Corynebacterium or Brevibacterium and that the gene responsible for the resistance could be expressed (Japanese Published Unexamined Patent Application No. 186492/82). Further, as described in the production of histidine of the present invention, it is apparent that the gene expressed in Corynebacterium glutamicum is expressible in broad host microorganisms of the genera Corynebacterium, Brevibacterium and the like. Therefore, all the glutamic acid-producing microorganisms including those belonging to the genus Corynebacterium or Brevibacterium as well as the species Corynebacterium glutamicum are competent as the host microorganism of the present invention.

For the strains appearing in the present specification, the accession numbers, deposition date and transfer date of the deposits under the Budapest Treaty of the International Recognition of the Deposit of Microorganisms for the purposes of Patent Procedure are as follows.

| | ATCC | FERM P (BP) | Deposition date (Transfer date) Year/Month/Day |
|---|---|---|---|
| Corynebacterium glutamicum L-15 | 31834 | | 1981. 3. 9 ( ) |
| Corynebacterium herculis L-103 | 31866 | | 1981. 4. 3 ( ) |
| Brevibacterium divaricatum L-204 | 31867 | | 1981. 4. 3 ( ) |
| Brevibacterium lactofermentum L-312 | 31868 | | 1981. 4. 3 ( ) |
| Corynebacterium glutamicum LA103/pCE54 | 39019 | | 1981.12.11 |
| Corynebacterium glutamicum LA103/pCB101 | 39020 | | 1981.12.11 |
| Corynebacterium glutamicum LA103/pEthr1 | 39021 | | 1981.12.11 |
| Corynebacterium glutamicum LA103/pCG11 | 39022 | | 1981.12.11 |
| Corynebacterium glutamicum K17 | 39032 | | 1981.12.21 |
| Corynebacterium glutamicum K18 | 39033 | | 1981.12.21 |
| Corynebacterium glutamicum K19 | 39034 | | 1981.12.21 |
| Corynebacterium glutamicum K20 | 39035 | | 1981.12.21 |
| Corynebacterium glutamicum K31 | 39280 | | 1983. 1.28 |
| Corynebacterium glutamicum K32 | 39281 | | 1983. 1.28 |
| Corynebacterium herculis K33 | 39282 | | 1983. 1.28 |
| Brevibacterium fluvum K34 | 39283 | | 1983. 1.28 |
| Brevibacterium lactofermentum K35 | 39284 | | 1983. 1.28 |

| | | |
|---|---|---|
| Corynebacterium glutamicum K37 | 39285 | 1983.1.31 |
| Corynebacterium glutamicum K36 | 6908 ( 451) | 1983. 2. 3 (1984. 1.19) |
| Corynebacterium glutamicum H33 | 6909 ( 452) | 1983. 2. 3 (1984. 1.19) |
| Corynebacterium glutamicum C156 | 6910 ( 453) | 1983. 2. 8 (1984. 1.19) |
| Corynebacterium glutamicum K38 | 7087 ( 454) | 1983. 5.19 (1984. 1.19) |
| Corynebacterium glutamicum K39 | 7088 ( 459) | 1983. 5.19 (1984. 1.21) |
| Corynebacterium glutamicum K40 | 7160 ( 455) | 1983. 7.21 (1984. 1.19) |
| Corynebacterium glutamicum K41 | 7161 ( 456) | 1983. 7.21 (1984. 1.19) |
| Corynebacterium glutamicum K43 | 7162 ( 457) | 1983. 7.21 (1984. 1.19) |
| Corynebacterium glutamicum K44 | 7163 ( 458) | 1983. 7.21 (1984. 1.19) |
| Corynebacterium glutamicum K45 | 7164 ( 460) | 1983. 7.21 (1984. 1.21) |
| Brevibacterium flavum K42 | ( 355) | 1983. 9.12 |
| Corynebacterium glutamicum K46 | ( 356) | 1983. 9.12 |
| Brevibacterium flavum K48 | ( 357) | 1983. 9.12 |
| Corynebacterium herculis K47 | ( 367) | 1983. 9.21 |
| Corynebacterium glutamicum K49 | ( 464) | 1984. 1.25 |

Example 1

**Preparation of DNA of the plasmid pCG11:**

pCG11 used as a vector plasmid was prepared from Corynebacterium glutamicum LA 103/pCG11, ATCC 39022 which is a derivative of Corynebacterium glutamicum L-22 and harbors pCG11 as follows.

The strain was grown with shaking at 30°C in 400 mℓ of NB medium to an OD value of about 0.7. Cells were harvested and washed with TES buffer. The cells were suspended in 10 mℓ of the aforementioned lysozyme solution and allowed to react at 37°C for 2 hours. Then 2.4 mℓ of 5M NaCl, 0.6 mℓ of 0.5M EDTA (pH 8.5) and 4.4 mℓ of a solution consisting of 4% sodium lauryl sulfate and 0.7M NaCl were added successively. The mixture was stirred slowly and allowed to stand on an ice water bath for 15 hours.

The whole lysate was centrifuged at 4°C under 69,400 x g for 60 minutes. The supernatant fluid was recovered and 10% (by weight) polyethyleneglycol (PEG) 6,000 (product of Nakarai Kagaku Yakuhin Co.) was added. The mixture was stirred slowly to dissolve completely and then kept on an ice water bath. After 10 hours, the mixture was subjected to centrifugation at 1,500 x g for 10 minutes to recover a pellet. The pellet was redissolved gently in 5 mℓ of TES buffer and 2.0 mℓ of 1.5 mg/mℓ ethidium bromide was added. Then, cesium chloride was added to adjust the density of the mixture to 1.580. The solution was centrifuged at 18°C at 105,000 g for 48 hours. After the density gradient centrifugation, a covalently-closed circular DNA was detected under UV irradiation as a high density band located in the lower part of the centrifugation tube. The band was taken out from the side of the tube with an injector to obtain a fraction containing pCG11 DNA. To remove ethidium bromide, the fraction was treated five times with an equal amount of cesium chloride saturated isopropyl alcohol solution consisting of 90% by volume isopropyl alcohol and 10% TES buffer solution. Then, the residue was dialysed against TES buffer solution to obtain pCG11 plasmid DNA.

Example 2

Cloning of the gene coding for chorismate mutase and prephenate dehydratase derived for the strain Corynebacterium glutamicum K38 and production of phenylalanine by the expression of the gene in Corynebacterium glutamicum:

1) Preparation of the chromosomal DNA and plasmid pCG11:

The chromosomal DNA of Corynebacterium glutamicum K38 FERM P-7087 was prepared by the following method:

A seed culture was inoculated into 400 mℓ of SSM. Culturing was carried out with shaking at 30°C. NB medium was used as seed culture. The optical density (OD) at 660 nm was monitored with a Tokyo Koden colorimeter and penicillin G was added at an OD value of 0.2 to a concentration of 0.5 unit/mℓ. Culturing was continued to an OD value of about 0.6.

Cells were harvested from the culture broth and washed with TES buffer. The cells were suspended in a lysozyme solution (pH 8.0) consisting of 25% sucrose, 0.1M NaCl, 0.05M Tris and 0.8 mg/mℓ lysozyme (the same lysozyme solution was used hereinafter) to make 10 mℓ of a suspension which was allowed to react at 37°C for 4 hours. High molecular chromosomal DNAs were isolated from the cells by the method of Saito et al.

Separately pCG11 used as a vector was prepared by the method described in Example 1.

2) Cloning of the gene responsible for the phenotype resistant to para-fluorophenylalanine (PFP):

In this step, 5 units of restriction enzyme BglII was added to 100 µℓ of a BglII reaction solution containing 3 µg of pCG11 plasmid DNA, and 5 units of BamHI was added to 100 µℓ of restriction enzyme BamHI reaction solution (pH 8.0) consisting of 10 mM Tris, 7 mM $MgCl_2$, 100 mM NaCl, 2 mM mercaptoethanol P and 0.01% bovine serum albumin (the same BamHI reaction solution was used hereinafter) and containing 9 µg of the chromosomal DNA. The mixtures were allowed to react at 37°C for 60 minutes and heated at 65°C for 10 minutes to stop the reactions. The reaction mixtures were mixed with each other. 40 µℓ of a T4 ligase buffer solution II, 40 µℓ of 5 mM ATP, 0.4 µℓ of T4 ligase and 120 µℓ of $H_2O$ were added. The mixture was allowed to react at 12°C for 16 hours.

3) Transformation with recombinant plasmids:

The above ligation mixture was provided for the following transformation. As the recipient for the transformation, Corynebacterium glutamicium L-15 ATCC 31834 was used. The seed culture of L-15 strain was inoculated into NB medium and culturing was carried out with shaking at 30°C. Cells were harvested at an OD value of 0.6. The cells were suspended at about $10^9$ cells/mℓ in RCGP medium (pH 7.6) containing 1 mg/mℓ lysozyme. The suspension was put in an L-tube and stirred slowly at 30°C for 5 hours to obtain

protoplasts.

Then 0.5 mℓ of the protoplast suspension was put in a small test tube and centrifuged at 2,500 x g for 5 minutes. The protoplasts were resuspended in 1 mℓ of TSMC buffer and again subjected to centrifugation and washing. The washed protoplasts were resuspended in 0.1 mℓ of TSMC buffer solution. 100 μℓ of a mixture (1 : 1 by volume) of a two-fold concentrated TSMC buffer and the ligated DNA mixture described above was added to the protoplast suspension. Then, 0.8 mℓ of a solution containing 20% PEG 6,000 in TSMC buffer solution was added to the mixture. After 3 minutes, 2 mℓ of RCGP medium (pH 7.2) was added and the mixture was centrifuged at 2,500 x g for 5 minutes. The supernatant fluid was removed and the protoplasts were suspended in 1 mℓ of RCGP medium. Then, 0.2 mℓ of the suspension was spread on RCGP agar medium containing 200 μg/mℓ spectinomycin and incubated at 30 °C for 7 days.

All the spectinomycin-resistant colonies formed on the selection plate were scraped, washed with physiological saline solution and centrifuged two times. The cells were spread on a minimal agar medium M1 containing 100 μg/mℓ spectinomycin and 0.5 mg/mℓ PFP and incubated at 30 °C for 2 days. The transformants which are resistant to spectinomycin and PFP were obtained from the colonies formed.

Plasmid DNAs were isolated from cells of these transformants. The plasmid pCS-CM1, recovered from one of the transformants was analyzed by digestion with various restriction endonucleases and analyzed by agarose gel electrophoresis. The analysis showed that a BamHI DNA fragment of about 9.4 Kb was inserted into the unique BglII cleavage site of pCG11 in PCS-CM1.

Furthermore, the same ligation mixtures was used to transform a Corynebacterium glutamicum strain requiring phenylalanine and tyrosine for its growth (defective in chorismate mutase and prephenate dehydratase) by the same method described above. Transformants resistant to spectinomycin and non-requiring phenylalanine and tyrosine were selected.

Plasmid DNAs were isolated from these transformants as mentioned above. A plasmid pCS-CM2 obtained from one of the transformants was digested with restriction endonucleases and analysed by agarose gel electrophoresis. The analysis showed that a BamHI DNA fragment of about 9.4 Kb was inserted into the unique BglII cleavage site of pCG11 in pCS-CM2.

Further, digestions with various restriction endonucleases such as EcoRI, SalI, HindIII and the like gave the same cleavage patterns in pCS-CM1 and pCS-CM2, indicating that both plasmids have the same physical structure as shown below.

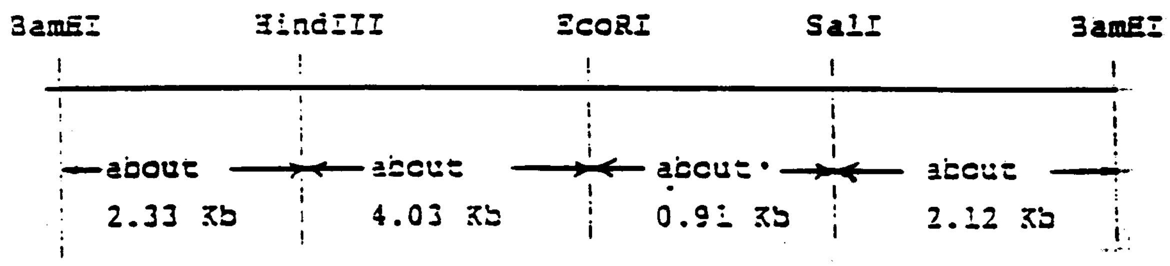


When a phenylalanine- and tyrosine-requiring Corynebacterium glutamicum stain was transformed with pCS-CM1 and pCS-CM2 by the same method mentioned above the resulting transformants grown on RCGP agar medium containing 100 μg/mℓ each phenylalanine and tyrosine and 200 μg/mℓ spectinomycin all exhibited phenylalanine- and tyrosine-prototrophy and resistance to PFP, and carried the same plasmids as pCS-CM1 and pCS-CM2.

These results indicate that the BamHI DNA fragment of about 9.4 Kb cloned in pCS-CM1 and pCS-CM2 contains the genes coding for chorismate mutase and prephenate dehydratase of Corynebacterium glutamicum K38 and that the DNA fragment can confer resistance to para-fluorophenylalanine on Corynebacterium glutamicum.

4) Production of phenylalanine by the transformant:

Corynebacterium glutamicum K38 (FERM P-7087, FERM BP-454) which produces phenylalanine was transformed with pCS-CM2 as mentioned above. The thus obtained transformant has been deposited with the Fermentation Research Institute as Corynebacterium glutamicum K39, FERM P-7088 (FERM BP-459).

Corynebacterium glutamicum K39, carrying pCS-CM2 was tested for L-phenylalanine production as follows.

The strain was cultured in NB medium at 30°C for 16 hours and 0.5 mℓ of the culture liquor was inoculated in 5 mℓ of a production medium P4 (pH 7.2) consisting of 100 g/l molasses, 20 g/l $(NH_4)_2SO_4$, 0.5 g/l $KH_2PO_4$, 0.5 g/l $K_2HPO_4$, 0.25 g/l $MgSO_4 \cdot 7H_2O$ and 20 g/l $CaCO_3$. Culturing was carried out at 30°C for 96 hours. The culture filtrate was subjected to paper chromatography and color reaction with ninhydrin. The amount of L-phenylalanine formed was determined colorimetrically. As a control, Corynebacterium glutamicum K38 was treated as mentioned above. The results are shown in Table I.

Table I

| Strain | Amount of L-phenylalanine (mg/mℓ) |
|---|---|
| Corynebacterium glutamicum K38 | 6.0 |
| Corynebacterium glutamicum K39 | 9.6 |

## Claims

1. A process for producing L-phenylalanine which comprises culturing a microorganism obtained by transforming a hoot microorganism belonging to the genus Corynebacterium or Brevibacterium with a recombinant DNA wherein a DNA fragment containing the gene coding for chorismate mutase and prephenate dehydratase is inserted into a vector DNA, accumulating L-phenylalanine in the culture and recovering L-phenylalanine therefrom.

2. The process according to claim 1, wherein the recombinant DNA is plasmid pCS-CM2 (FERM BP-459).

3. The process according to claim 1, wherein the microorganism is Corynebacterium glutamicum K39, FERM BP-459.

4. A biologically pure culture of Corynebacterium glutamicum K39, FERM BP-459.

## Patentansprüche

1. Verfahren zur Herstellung von L-Phenylalanin, umfassend die Züchtung eines Mikroorganismus, der erhalten wurde durch Transformation eines zur Gattung Corynebacterium oder Brevibacterium gehörenden Wirtsmikroorganismus mit einer rekombinanten DNA, in der ein das die Chorismatmutase und Prephenatdehydratase codierende Gen enthaltendes DNA-Fragment in eine Vektor-DNA eingefügt ist, die Anhäufung von L-Phenylalanin im Medium und seine Gewinnung daraus.

2. Verfahren nach Anspruch 1, wobei die rekombinante DNA das Plasmid pCS-CM2 (FERM BP459) ist.

3. Verfahren nach Anspruch 1, wobei der Mikroorganismus Corynebacterium glutamicum K39, FERM BP-459 ist.

4. Biologisch reine Kultur von Corynebacterium glutamicum K39, FERM BP-459.

## Revendications

1. Procédé de production de L-phénylalanine, caractérisé en ce que l'on cultive un micro-organisme obtenu par la transformation d'un micro-organisme hôte appartenant au genre Corynebacterium ou Brevibacterium à l'aide d'un ADN recombinant, dans lequel un fragment d'ADN contenant le gène codant pour la chorismate mutase et la préphénate déshydratase est inséré dans un ADN vecteur, l'accumulation de la L-phénylalanine dans la culture et la récupération de la L-phénylalanine dans cette culture.

2. Procédé suivant la revendication 1, caractérisé en ce que l'ADN recombinant est le plasmide pCS-CM2 (FERM BP-459).

**3.** Procédé suivant la revendication 1, caractérisé en ce que le micro-organisme est Corynebacterium glutamicum K39, FERM BP-459.

**4.** Culture biologiquement pure de Corynebacterium glutamicum K39, FERM BP-459.